# EUROPÄISCHE PATENTSCHRIFT

(11) **EP 3 130 330 B1**
(45) Veröffentlichungstag und Bekanntmachung des Hinweises auf die Patenterteilung: **26.09.2018**
(21) Anmeldenummer: 16173513.9
(22) Anmeldetag: 08.06.2016
(51) Int. Cl.: A61K 8/42, A61K 8/92, A61Q 19/10, A61Q 19/00, A61K 8/25

(54) **REINIGUNGSPRODUKT AUF ÖLBASIS MIT PARTIKELN**
OIL BASED CLEANING PRODUCT WITH PARTICULATES
PRODUIT DE NETTOYAGE BASÉE D'HUILE AVEC PARTICULES

(30) Priorität: 12.08.2015 DE 102015215359
(43) Veröffentlichungstag der Anmeldung: 15.02.2017
(73) Patentinhaber: Beiersdorf AG, 20253 Hamburg (DE)
(72) Erfinder: COUNRADI, Katrin, 22143 Hamburg (DE); KÖNIG, Sylvia, 21635 Jork (DE)

(56) Entgegenhaltungen:
- EP-A2- 0 557 825
- EP-A2- 1 955 690
- DE-A1- 10 253 304

## Beschreibung

Die vorliegende Erfindung umfasst Reinigungsprodukte auf Ölbasis in halbfester Form enthaltend Peeling-Partikel, insbesondere selbstauflösende Peelingpartikel.

Die Reinigung der Haut und Hautanhangsgebilde gehört heutzutage in vielen Kulturkreisen zum täglichen Leben, fast vergleichbar wie Essen und Trinken. Bei der Reinigung wird in erster Linie Schmutz, der häufig in Form eines Films auf der Hautoberfläche liegt, von der Hautoberfläche entfernt. Der Schmutzfilm besteht aus festen oder flüssigen Komponenten, die von außen auf die Haut gelangt sind. Ebenfalls zum Schmutzfilm gehören überschüssige Hautlipide und abgestorbene Körperzellen. Durch oberflächenaktive Inhaltsstoffe in den Reinigungszusammensetzungen gelangen die Komponenten des Schmutzfilms in die Waschflotte und werden durch den Abspülprozess von der Haut entfernt. Neben der Beseitigung des Schmutzfilms können jedoch auch, je nach den Reinigungsbedingungen mehr oder weniger, Oberflächenlipide der Haut, beispielsweise Barrierelipide, durch die oberflächenaktiven Substanzen in den Reinigungszubereitungen gelöst und im Abspülvorgang mit entfernt werden. Der Verlust der Hautlipide kann zu Austrocknungserscheinungen und Hautirritationen führen.

Diese unerwünschten Auswirkungen sind besonders bei Menschen mit trockener Haut zu beobachten. Trockene Haut zeichnet sich durch einen Mangel an Feuchtigkeit und Fettstoffen in der obersten Hautschicht aus. Die Haut ist rau, schuppig, glanzlos und wenig elastisch. Es wird häufig ein unangenehmes Spannungsgefühl oder ein Juckreiz empfunden, der insbesondere nach dem Waschen auftreten kann. Dieser Juckreiz führt dazu, dass der Betroffene sich häufig kratzt. Dies wiederum führt dann zu kleinen Verletzungen, die ein Einfallstor für Krankheitserreger sein können. Ebenso können Hautentzündungen auftreten, die sich in Form von Rötungen, Schuppungen, Bläschen und Pusteln ausbilden. Neben den sichtbaren und spürbaren Symptomen lässt sich trockene Haut auch durch Messungen der Rauigkeit, des Wasserverlustes, des Wassergehaltes und des Fettes charakterisieren. Trockene Haut kann erblich bedingt sein und zum Beispiel durch falsche Pflegegewohnheiten, Flüssigkeitsmangel, einseitige Ernährung, Hormonschwankungen und psychische Belastungen verstärkt werden. Aber auch andere Krankheiten können zum Auftreten von trockener Haut führen. Zu nennen sind hier zum einen Hautkrankheiten, wie beispielsweise Neurodermitis, atopische Dermitis, Schuppenflechte und Ichtyosen und zum anderen Stoffwechselerkrankungen wie beispielsweise Schilddrüsenunterfunktion und Diabetes.

Bei der Reinigung trockener Haut sollte beachtet werden, der Haut so wenig wie möglich Fette und Feuchtigkeit zu entziehen. Neben der Beachtung der allgemeinen Regel "so viel wie nötig, so wenig wie möglich", die sich auf die Häufigkeit der Reinigung, insbesondere in Form von Duschbädern oder Wannenbädern bezieht, sollten die Reinigungsprodukte so ausgewählt werden, dass sie besonders mild sind, um die Haut nicht zu reizen und/oder sich durch einen hinreichenden Gehalt an rückfettenden Substanzen auszeichnen.

Besonders geeignet für Menschen mit trockner Haut ist die Anwendung einer Reinigungszubereitung in Form eines Ölbades. Hier können drei Klassen unterschieden werden. Zum einen gibt es Zubereitungen, die im Wesentlichen aus Ölen und Duftstoffen bestehen. Diese Produkte bilden einen Film auf der Wasseroberfläche, der beim Verlassen des Bades auf die Haut aufzieht. In der Regel schäumen derartige Produkt nicht und entfalten auch keine reinigende Wirkung. Der pflegende Aspekt steht bei diesen Produkten im Vordergrund.

Es ist auch möglich, Öl-haltige Badezubereitungen mit Emulgatoren zu versetzen. Bei einer verhältnismäßig geringen Menge an Emulgator tritt eine Trübung bei der Zugabe ins Badewasser auf. Es ist möglich die Emulgatormenge so weit zu erhöhen, dass die Trübung vermieden werden kann.

In einer weiteren Zubereitungsform ölhaltiger Badezubereitungen werden waschaktive Substanzen zugesetzt. Dieser Zusatz führt dazu, dass eine Schaumbildung erfolgen kann. Im Allgemeinen bedeutet der Zusatz dieser waschaktiven Substanzen jedoch, dass die Pflegeleistung abnimmt.

Öl-haltige Badezubereitungen sind an sich bekannt, wie der Stand der Technik zeigt.

Das Dokument DE 29 43 202 beschreibt Mittel mit reinigender und hautpflegender Wirkung auf der Basis von Gemischen aus Tensiden und Ölen, die bevorzugt als sogenannte Pflegeschaumbäder eingesetzt werden, wobei allerdings auch die Verwendung dieser Mittel als Duschzubereitung erwähnt wird. Die beschriebenen Zubereitungen haben einen Gehalt von 20 bis 80 Gew.-% einer wässrigen Tensidlösung, die ihrerseits aus 85 bis 95 Gew.-% Tensid und 5 bis 15 Gew.-% Wasser besteht, sowie einen Ölgehalt von 80 bis 20 Gew.-%. Die waschaktive Komponente dieser Zubereitungen besteht aus Mono-oder Dialkylamin-, Mono-oder Dialkanolamin-oder Alkylalkanolaminsalzen von Fettalkoholschwefelsäureestern.

Die Schrift EP 120 224 beschreibt wirkstoffhaltige Ölbadzubereitungen mit einem Gehalt an 38,75 Gew.-% Sojaöl, 2,00 Gew.-% Rizinusöl, 37,00 Gew.-% Vaselineöl. Als Emulgator werden Polyethylenglycolmono- bzw. -diester offenbart, in Konzentrationen von ca. 10-12 Gew.-%. Der eingesetzte Wirkstoff ist Pelargonsäure.

Das Dokument DD 236014 offenbart ölhaltige kosmetische Mittel, die klar sein müssen. Diese Mittel finden u.a. Verwendung als Badeöle und Cremebäder. Neben 20 bis 80 % Öl, einer wässrigen Aniontensidlösung oder Wasser enthalten diese Zubereitungen eine Kombination aus bestimmten hydrophoben und hydrophilen Emulgatoren bzw. nichtionogenen Tensiden.

In dem Dokument DE 44 24 210 werden kosmetische oder dermatologische Duschzubereitungen mit einem Tensidgehalt von höchstens 55 Gew.-% und einem Ölgehalt von mehr als 45 Gew.-% beschrieben, wobei die Zubereitungen im Wesentlichen wasserfrei sind. Aufgrund des hohen Ölgehalts wirken diese Zubereitungen regenerierend in Bezug auf den allgemeinen Hautzustand. Sie haben dabei gleichzeitig eine gute Schaumentwicklung und eine hohe Reinigungskraft.

Die Schrift EP 0867176 beschreibt Duschzubereitungen, die einen hohen, jedoch im Vergleich zum Stand der Technik deutlich geringeren, Ölgehalt aufweisen. Es wird aber dennoch eine hohe Rückfettung erzielt bei besserer Schaumleistung und Verträglichkeit.

Das Dokument WO 03/051319 offenbart ebenfalls ölhaltige Reinigungsprodukte, in diesem Fall jedoch Reinigungsprodukte auf der Basis von ölhaltigen Mikroemulsionen. Es wird ein Verfahren zur Herstellung dieser ölhaltigen Mikroemulsionen beschrieben. Bei einer konstanten Gesamttensidmenge kann das Primärtensid-/Cotensid-Verhältnis variiert werden.

In dem Dokument WO 2005/065629 wird offenbart, dass ölhaltige, wasserfreie Tensidzubereitungen derart ausgestaltet werden können, dass sie einer Enzymschädigung der Enzyme der oberen Hautschichten entgegenwirken.

Im Dokument WO 2012/104025 werden auch lipidhaltige Badezubereitungen beschrieben, diese werden mit probiotisch aktiven Wirkstoffen versetzt.

Das Dokument DE 10253304 A1 offenbart kosmetische Masken, die den Einsatz von Ölen und auch chemisch modifizierten Fetten, wie beispielsweise hydrierten Pflanzenölen, beschreiben. Es handelt sich um Polyol-in-ÖI oder Öi-in-Polyol-Emulsionen.

DE 19843547 A1 beschreibt Duschöle, die Öle, Lecithin und Tenside in Form einer Mischung aus ausgewählten Tensiden enthalten. Es handelt sich um flüssige Zubereitungen, die keine Gele bilden.

Ebenso sind in der Mintel-Datenbank ölhaltige Reinigungszubereitungen zu finden. Beispielhaft seien hier folgende Beispiele erwähnt:
Kneipp Duschöl Schönheitsgeheimnis, Mintel-Nummer 2721675; dieses Duschöl enthält vier natürliche Öle, es wirkt rückfettend und feuchtigkeitsspendend.

Rossmann Wellness&Beauty Duschöl mit Mandelöl und Bambusextrakt, Mintel-Nummer 2303447; dieses Duschöl enthält ebenfalls natürliche Öle und schützt die Haut vor dem Austrocknen.

CD Pflege Duschöl, Mintel-Nummer 1715513; in diesem Duschöl ist Avocadoöl enthalten, das Duschöl reinigt die Haut schonend und schützt vor trockener und empfindlicher Haut.

Die Anwendung ölhaltiger Reinigungszubereitungen ist bei Menschen, die eine trockene oder empfindliche Haut oder Altershaut haben, sinnvoll und erwünscht. Darüber hinaus kommt auch bei dieser Personengruppe der Wunsch auf, geeignete Produkte zur Hauterfrischung und tiefergehenden Reinigung zu haben oder Produkte einsetzen zu können, die die Durchblutung der Haut auf sanfte Weise zu fördern vermögen, ohne die Haut anzugreifen, zu beeinträchtigen oder zu reizen.

Die Lösung liegt in der Bereitstellung von Reinigungsmitteln, die einen Peeling-Effekt aufweisen. Die peelende Wirkung kann dabei durch den Einsatz abrasiver Partikel zustande kommen. Bei den abrasiven Partikel handelt es sich in der Regel um natürliche oder synthetische, wasserunlösliche Feststoffe, in Form von Partikeln, die einen durchschnittlichen Durchmesser von 100 bis 400 µm und eine runde oder unrunde Gestalt aufweisen.

Im Rahmen einer Peelingbehandlung kann die abrasive Wirkung eingesetzter Partikel die Haut reizen. In Ausnahmefällen, insbesondere bei trockener und/oder empfindlicher Haut und/oder Altershaut kann es dadurch zu Irritationen kommen.

In der Vergangenheit hat es nicht an Versuchen gefehlt, dieser Reizung entgegen zu wirken, z. B. durch den Einsatz von Peelingpartikeln, die sich bei der Benutzung auflösen und somit einen abnehmenden Peelingeffekt zeigen.

Wasserlösliche Partikel, wie z. B. aus kristallbildenden Zuckern oder Salzen, können in mit diesen Zuckern oder Salzen übersättigte wässrige tensidhaltige Lösungen integriert werden. Derartige Lösungen sind aber schwer zu stabilisieren; die Partikel neigen dazu sich abzusetzen.

Unter dem Begriff Zucker können Mono- und Oligosaccharide (Dimere bis Decamere von Monosacchariden) verstanden werden, die süß schmecken, wasserlösliche und meist kristalline Verbindungen sind. Dem stehen die Polysaccharide gegenüber, die nur schlecht oder gar nicht in Wasser löslich sind, keine einheitliche Molmasse haben und praktisch geschmacksfrei sind.

Reinigungszubereitungen, die Peelingpartikel enthalten, die sich in Verlaufe der Anwendung auflösen, werden beispielsweise in den Dokumenten DE 102013224336 und DE 102013224368 beschrieben. Diese Zubereitungen sind Zubereitungen auf wässriger Basis, die keine oder nur geringe Mengen an Lipiden enthalten.

Die ölhaltigen Zubereitungen des Standes der Technik haben jedoch Nachteile. Sie sind dünnflüssig, d.h. sie haben in der Regel Viskositätswerte < 1000 mPa·s. Dies führt dazu, dass bei Anwendung derartiger Zubereitungen als Duschzubereitung ein Großteil der Zubereitung leicht von der Hand fließen kann, bevor eine Verteilung auf dem Körper möglich ist. Der Teil der Duschzubereitung, der nicht auf dem Körper verteilt werden konnte, verteilt sich in der Regel auf dem Boden der Duschkabine und kann dazu führen, dass der Boden der Duschkabine rutschig und glitschig wird. Dann besteht durch Ausrutschen eine Verletzungsgefahr.

Zudem kann bei der Anwendung derartiger Produkt beobachtet werden, dass auch die Hände, auf die derartige Produkte aufgebracht wurden, glitschig werden. Bei älteren Badezimmerarmaturen, bei denen ein Nachregeln der Wassertemperatur durch Drehen der entsprechenden Wasserhähne erfolgen muss, führt dies zu Problemen. Das Nachregeln der Temperatur ist erschwert.

Wünschenswert sind also ölhaltige Reinigungszubereitungen, insbesondere Duschzubereitungen, die weniger dünnflüssig sind und zugleich eine sanfte Peelingwirkung aufweisen. Im Stand der Technik werden Zubereitungen offenbart, die diesem Nachteil wenigstens zum Teil entgegenwirken. Im Dokument DE 10350425 wird ein zähflüssiges Duschöl beschrieben, dem bestimmte Verdicker zugesetzt werden. Diese Verdicker sind Polyamid-Gelbildner, die jedoch dazu führen, dass die Sensorik von Zubereitungen enthaltend diesen Verdicker als negativ empfunden wird. Häufig sind derartige Zubereitungen fadenziehend und klebend.

Die Aufgabe der vorliegenden Erfindung war es also ölhaltige Reinigungszubereitungen zur Verfügung zu stellen, deren Anwendung nicht zu den beschriebenen Nachteilen führt, insbesondere ölhaltige Reinigungszubereitungen zur Verfügung zu stellen, die Viskositätswerte > 5000 mPa·s aufweisen. Zugleich sollten die Reinigungszubereitungen eine sanfte Peelingwirkung entfalten, ohne die Haut zu reizen oder anzugreifen.

Darüber hinaus sollten die Zubereitungen aus möglichst wenigen, weitgehend natürlichen oder Natur-basierten Komponenten bestehen und frei sein von üblicherweise verwendeten polymeren Strukturanten und frei sein von Polyamid-Gelbildnern.

Diese Reinigungszubereitungen sollten eine gute Reinigungsleistung zugleich aber eine sehr gute Pflegeleistung erbringen und für alle Hauttypen geeignet sein, insbesondere jedoch für trockene Haut. Nach der Anwendung der erfindungsgemäßen Produkte sollte es nicht zu Spannungsgefühlen der Haut oder Reizungen der Haut kommen, wie beispielsweise deutliche Rötungen oder Juckreiz.

Überraschend wurden diese Aufgaben gelöst durch
ölhaltige Reinigungszubereitungen in halbfester Form mit abrasiver Wirkung enthaltend
a. Wasser in einer Menge < 1,5 Gew.-%, bevorzugt < 0,2 Gew.-%, bezogen auf das Gesamtgewicht der Zubereitung,
b. Polyole mit einem Gehalt von < 10 Gew.-%, bezogen auf das Gesamtgewicht der Zubereitung,
c. Lipide, wobei die Lipide Gemische von wenigstens einem bei Raumtemperatur in flüssiger Form vorliegenden Lipid und von wenigstens einem bei Raumtemperatur in fester Form vorliegenden Lipid sind,
   wobei das Gewichtsverhältnis des wenigstens einen bei Raumtemperatur festen Lipids zu dem wenigstens einen bei Raumtemperatur flüssigen Lipids 1:2 bis 7:1, bevorzugt 1:1 bis 3:1 beträgt,
   wobei die bei Raumtemperatur in fester Form vorliegenden Lipide aus gehärteten Fetten und/oder Ölen bestehen und
   die bei Raumtemperatur flüssigen Lipide pflanzliche Öle sind,
d. Gemische aus MIPA-Laurethsulfat, Laureth-4 und Kokosfettsäurediethanolamid als öllösliche Tenside,
e. abrasive Partikel, wobei die abrasiven Partikel feste Partikel sind, die lösliche oder unlösliche Partikel sind.

In besonders vorteilhaften Ausführungsformen wird den erfindungsgemäßen Zubereitungen kein Wasser zugesetzt und/oder über Zubereitungskomponenten eingeschleppt. Ganz besonders vorteilhaft sind erfindungsgemäße Zubereitungen, die kein Wasser enthalten.

Die bei Raumtemperatur flüssigen Lipide sind pflanzliche Öle. Es ist bevorzugt, wenn die flüssigen Lipide aus der Gruppe Rizinusöl, Sojaöl und Öl aus Sonnenblumenkernen ausgewählt werden. In den erfindungsgemäßen Zubereitungen ist wenigstens ein flüssiges Lipid vorhanden.

Die bei Raumtemperatur infester Form vorliegenden Lipide bestehen aus gehärteten Ölen und/oder Fetten. Es ist bevorzugt, die festen Lipide aus der Gruppe Hydrogenated Vegetable Oil und Hydrogenated Rapeseed Oil auszuwählen. Hydrogenated Vegetable Oil ist beispielsweise erhältlich bei der Firma Dr. Straetmans unter der Handelsbezeichnung Dermofeel Viscolid oder bei der Firma AAK unter der Handelsbezeichnung Akogel Plus. Hydrogenated Rapeseed Oil ist beispielsweise erhältlich bei der Firma ADM unter der Handelsbezeichnung VGB 22. In den erfindungsgemäßen Zubereitungen ist wenigstens ein festes Lipid vorhanden.

Die bei Raumtemperatur festen und flüssigen Lipide liegen in einem bestimmten Verhältnis zueinander vor, um die gewünschte Konsistenz der Zubereitung zu erzielen. Als vorteilhaft haben sich die Gewichtsverhältnisse der bei Raumtemperatur festen Lipide zu den bei Raumtemperatur flüssigen Lipiden von 1:2 bis 7:1, bevorzugt 1:1 bis 3:1 erwiesen.

Die Gemische von wenigstens einem bei Raumtemperatur in flüssiger Form vorliegenden Lipid und von wenigstens einem bei Raumtemperatur in fester Form vorliegenden Lipid liegen einem Gehalt von 20 bis 60 Gew.-%, bevorzugt 25 bis 40 Gew.-%, bezogen auf das Gesamtgewicht der Zubereitung, vor.

Es ist bevorzugt, wenn die Lipide und die öllöslichen Tenside in einem bestimmten Gewichtsverhältnis vorliegen; nämlich in einem Gewichtsverhältnis von Lipiden zu öllöslichen Tensiden von 1: 3 bis 2:1.

Die Reinigungszubereitungen enthalten als öllöslicheTenside Gemische aus MIPA-Laurethsulfat, Laureth-4 und Kokosfettsäurediethanolamid.

Der Gehalt an öllöslichen Tensiden beträgt 10 bis 60 Gew.-%, bevorzugt 20 bis 50 Gew.-%, insbesondere bevorzugt 25 bis 40 Gew.-%, bezogen auf das Gesamtgewicht der Zubereitung.

Die Reinigungszubereitungen enthalten < 5,0 Gew. %, bevorzugt < 1,5 Gew. %, besonders bevorzugt < 0,2 Gew. % Wasser. Der Wassergehalt kann mit Hilfe der Karl-Fischer-Titration bestimmt werden. Die Wasserbestimmung nach Karl Fischer ist geeignet um den Wassergehalt in organischen Lösungsmitteln, ätherischen Ölen, Salben und anderen organischen Substanzen zu bestimmen. Grundlage des Verfahrens ist die Tatsache, dass Jod und Schwefeldioxid nur in Gegenwart von Wasser zu Jodid und Sulfat reagieren. Der Endpunkt der Titration wird durch das Auftreten einer gelb-braunen Färbung angezeigt; an diesem Punkt ist das gesamte Wasser verbraucht und Jod reagiert nicht mehr zum farblosen Jodid. Heute wird diese Bestimmung mit vorbereiteten, käuflich zu erwerbenden Lösungen durchgeführt.

In den erfindungsgemäßen Reinigungszubereitungen sind als feste abrasive Partikel lösliche und/oder unlösliche Partikel enthalten.

Im Sinne der vorliegenden Erfindung werden unter festen abrasiven Partikeln, die löslich sind, Partikel verstanden, die in der Reinigungszubereitung im festen Aggregatzustand, insbesondere in Kristallform, vorliegen. Bei Anwendung der Reinigungszubereitung, beispielsweise beim Duschen, kommt die Reinigungszubereitung mit einer größeren Menge Wasser in Kontakt, so dass die Reinigungszubereitung verdünnt wird. Dies führt in der Regel dazu, dass sich die festen Partikel, insbesondere Kristalle, allmählich auflösen. Dies sind dann selbstauflösende Partikel.

Die festen abrasiven Partikel, die löslich sind, werden bevorzugt aus Zuckern und/oder geeigneten und hautverträglichen Salzen generiert. Besonders bevorzugt ist der Einsatz von Saccharose und/oder Natriumchlorid zur Ausbildung der löslichen Partikel.

Die erfindungsgemäß einsetzbaren festen abrasiven Partikel, die unlöslich sind, haben einen synthetischen und/oder natürlichen Ursprung. Unlösliche Partikel natürlichen Ursprungs können sowohl mineralischen als auch pflanzlichen Ursprungs sein.

Die erfindungsgemäßen Reinigungszubereitungen können feste abrasive Partikel, die löslich sind, oder feste abrasive Partikel, die unlöslich sind, enthalten, sowie auch Mischungen löslicher und unlöslicher Partikel.

Es ist nicht erforderlich, den erfindungsgemäßen Reinigungszubereitungen polymere Strukturanten zuzusetzen. Unter polymeren Strukturanten werden Polymere verstanden, die sich aus einer Vielzahl chemisch einheitlich aufgebauter Struktureinheiten zusammensetzen und synthetischer Natur sind. Im Sinne der vorliegenden Erfindung werden Komponenten, die sich wiederholende Alkoxy-Einheiten, insbesondere Ethoxy-Einheiten, enthalten, nicht als polymere Strukturanten verstanden. Polymere Strukturanten im Sinne der vorliegenden Erfindung sind insbesondere diejenigen Polymere, die als Struktureinheit (Monomer) wenigstens Acrylsäure und/oder Methacrylsäure und/oder Derivate der Acrylsäure und/oder Methacrylsäure enthalten. Die erfindungsgemäßen Zubereitungen sind daher frei von polymeren Strukturanten.

Als Polyamidgelbildner werden Substanzen der folgenden chemischen Zusammensetzung verstanden: 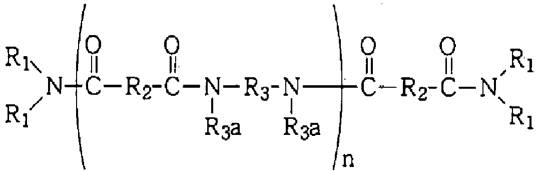 wobei
n = 0-5,
R₁ = Kohlenwasserstoff mit 1-22 Kohlenstoffatomen,
R₂ = Kohlenwasserstoff mit 2-42 Kohlenstoffatomen,
R₃ = Kohlenwasserstoff mit 2-36 Kohlenstoffatomen, enthaltend 0-3 Heteroatome, gewählt aus der Gruppe Sauerstoff und Stickstoff,
R₃^{a} = H, C1 -C10 -Kohlenstoffrest bedeuten. Die erfindungsgemäßen Reinigungszubereitungen sind frei von Polyamidgelbildnern.

Die erfindungsgemäßen Reinigungszubereitungen zeichnen sich dadurch aus, dass der Zusatz von Konservierungsmitteln nicht erforderlich ist. Daher sind die erfindungsgemäßen Reinigungszubereitungen frei von Konservierungsmitteln.

Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Um die Wirkung von Konservierungsstoffen zu ergänzen oder zu verstärken, werden kosmetischen Zubereitungen Substanzen zugesetzt, die die Wirkung von Konservierungsmitten verstärken, so dass es möglich ist, die Konzentration des Konservierungsmittels abzusenken. Diese Substanzen werden im Folgenden als verstärkende Substanzen bezeichnet.

Verstärkende Substanzen sind beispielsweise folgende Verbindungen: Alcohol Denat., Ethylhexylglycerin, 1,2-Hexanediol, Trisodium EDTA, Methylpropanediol, Butylene Glycol, Alcohol Denat., Ethylhexylglycerin, 1,2-Hexanediol, Polyaminopropyl Biguanide, Trisodium EDTA und Caprylyl Glycol.

Die erfindungsgemäßen Zubereitungen sind frei von den verstärkenden Substanzen.

Gleichwohl ist es denkbar, in alternativen Ausführungsformen Konservierungsmittel und/oder die verstärkenden Substanzen einzusetzen.

Den erfindungsgemäßen Reinigungszubereitungen werden keine Farbstoffe zugesetzt. Die erfindungsgemäßen Zubereitungen sind also frei von Farbstoffen. Gleichwohl ist es denkbar, in alternativen Ausführungsformen Farbstoffe einzusetzen.

Der pH-Wert der erfindungsgemäßen Reinigungszubereitungen kann durch den Zusatz von Milchsäure oder Zitronensäure eingestellt werden, wobei Zitronensäure bevorzugt ist. Wenn der Zusatz von Zitronensäure erforderlich ist, liegt Zitronensäure in den erfindungsgemäßen Zubereitungen in einer Konzentration von 0,01 bis 0,2 Gew.-%, bevorzugt mit 0,02 bis 0,15 Gew.-% vor, bezogen auf das Gesamtgewicht der Zubereitung.

Die erfindungsgemäßen Reinigungsprodukte sind überaus vorteilhafte Zubereitungen, die ohne den Einsatz von polymeren Strukturanten, Polyamidgelbildnern, Konservierungsmitteln und Farbstoffen auskommen und darüber hinaus aus wenigen, weitgehend natürlichen oder Natur-basierten Rohstoffen zusammengesetzt sind. Daher sind die erfindungsgemäßen Reinigungszubereitungen überaus geeignet zur tiefergehenden und erfrischenden Reinigung bei trockener und/oder empfindlicher Haut und/oder Altershaut, insbesondere jedoch bei trockener Haut.

Vorteilhaft ist daher die Verwendung von erfindungsgemäßen Reinigungszubereitungen zur tiefergehenden und erfrischenden Reinigung bei trockener und/oder empfindlicher Haut und/oder Altershaut, insbesondere trockener Haut.

Ebenso vorteilhaft sind ölhaltige Reinigungszubereitungen in halbfester Form mit abrasiver Wirkung enthaltend
a. Wasser in einer Menge < 5,0 Gew.-%, bevorzugt < 1,5 Gew.-%, besonders bevorzugt < 0,2 Gew.-%, bezogen auf das Gesamtgewicht der Zubereitung,
b. Polyole mit einem Gehalt von < 10 Gew.-%, bezogen auf das Gesamtgewicht der Zubereitung,
c. Lipide, wobei die Lipide Gemische von wenigstens einem bei Raumtemperatur in flüssiger Form vorliegenden Lipid und von wenigstens einem bei Raumtemperatur in fester Form vorliegenden Lipid sind,
d. öllösliche Tenside und
e. abrasive Partikel
zur Förderung der Durchblutung der Haut.

Bei der Förderung der Durchblutung der Haut erlangt die Haut ein rosiges Erscheinungsbild, das nicht verwechselt werden darf mit Rötungen der Haut, die nicht erwünscht sind und die es zu vermeiden gilt.

Die erfindungsgemäßen Reinigungszubereitungen sind vorteilhaft zu verwenden als Duschzubereitungen. Ebenso vorteilhaft ist die Verwendung als Gesichtsreinigungsprodukt. Denkbar sind auch andere Anwendungsformen, beispielsweise Handwaschmittel oder Shampoo.

Das erfindungsgemäße Fettalkoholethersulfat ist MIPA-Laurethsulfat.

Die vorteilhaften Fettalkoholethoxylate weisen folgende Struktur auf:

R³-(O-CH₂-CH₂-)_{c}-OH

Dabei kann c Werte von 2 bis 10 annehmen, bevorzugt von 2 bis 6. R³ wird gewählt aus der Gruppe der verzweigten und unverzweigten Alkylgruppen mit 8 bis 16 Kohlenstoffatomen, insbesondere 10 bis 14 Kohlenstoffatome.

Erfindungsgemäßes Fettalkoholethoxylat ist Laureth-4.

Vorteilhafte Fettsäuremono- bzw. -diethanolamide weisen folgende Strukturen auf:

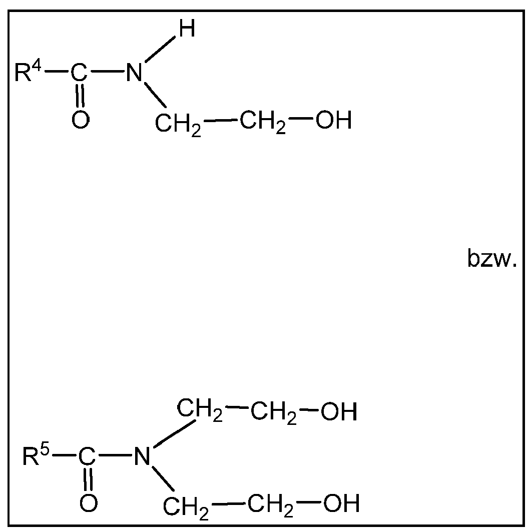

R⁴ bzw. R⁵ werden dabei gewählt aus der Gruppe der verzweigten und unverzweigten Alkylgruppen und/oder Alkenylgruppen mit 6 bis 24 Kohlenstoffatomen, insbesondere mit 10 bis 16 Kohlenstoffatomen.

Das erfindungsgemäße Fettsäurediethanolamid ist Kokosfettsäurediethanolamid (Cocamide DEA). Natürliche Kokosfettsäure enthält als wesentliche Bestandteile Laurinsäure zu 44-51 Gew.-%, Myristinsäure zu 13-18 Gew.-%, Palmitinsäure zu 8-10 Gew.-%, Caprylsäure zu 6-9 Gew.-%, Caprinsäure zu 6-10 Gew.-%, Ölsäure zu 5- 8 Gew.-%, Stearinsäure zu 1-3 Gew.-%, Linolsäure zu 0-2 Gew.-% und Capronsäure zu 0-1 Gew.-%.

Gemische aus MIPA-Laurethsulfat, Laureth-4 und Kokosfettsäurediethanolamid sind als öllösliche Tenside einzusetzen. Solche Gemische sind beispielsweise unter der Bezeichnung ZETESOL® 100 von der Firma Zschimmer & Schwarz Chemische Fabriken, Lahnstein/Rhein, oder TEXAPON® WW 99 von der Henkel KGaA, Düsseldorf, erhältlich.

Die erfindungsgemäßen *Öle* sind pflanzliche Öle, also Triglyceride folgender Struktur: 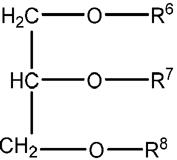 wobei R⁶, R⁷ und R⁸ unabhängig voneinander gewählt werden aus der Gruppe der verzweigten und unverzweigten, Alkylcarboxyl bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Für die Auswahl der bei Raumtemperatur flüssigen Lipide ist es insbesondere vorteilhaft, wenn R⁶, R⁷ und/oder R⁸ 16 bis 20 Kohlenstoffatome aufweisen und aus der Gruppe der einfach bis dreifach ungesättigten Carbonsäurereste gewählt werden.

Wenn R⁶, R⁷ und/oder R⁸ Hydroxylgruppen tragen, ist der bevorzugte Alkenylcarboxylrest der Ricinolsäurerest.

Besonders vorteilhaft ist, die erfindungsgemäßen bei Raumtemperatur flüssigen Lipide aus der Gruppe Sojaöl, Rizinusöl und Sonnenblumenkernöl auszuwählen.

Für die Auswahl der bei Raumtemperatur festen Lipide ist es vorteilhaft, wenn diese einen Schmelzpunkt oder Schmelzbereich in einem Fenster von 50 bis 70°C, eine Jodzahl zwischen 0 und 5 und einen Gehalt an freien Fettsäuren von 0 bis 0,8 Gew.-% haben.

Polyole in Sinne der vorliegenden Erfindung sind mehrwertige Alkohole. Es handelt sich um niedermolekulare Verbindungen, die mindestens zwei Hydroxylgruppen tragen. Erfindungsgemäß bevorzugt sind die Verbindungen Propylenglycol und Glycerin, insbesondere bevorzugt ist Glycerin. Der Gehalt an Polyolen beträgt 0 bis 10 Gew. %, bevorzugt 0 - 6,0 Gew. % beträgt, bezogen auf das Gesamtgewicht der Zubereitung beträgt. Es gibt also auch vorteilhafte erfindungsgemäße Ausführungsformen, die keine Polyole enthalten.

Feste abrasive Partikel in Sinne der vorliegenden Erfindung sind lösliche und/oder unlösliche Partikel, die eine Peelingwirkung hervorrufen.

Bei den löslichen Partikeln wird die Peelingwirkung durch den ungelösten Anteil an Zuckern oder Salzen, der in kristalliner Form vorliegen kann, hervorgerufen. Dies wird durch eine so hohe Zucker- und/oder Salzkonzentration erreicht, dass sich nicht alle Zucker- und/oder Salzmoleküle in der erfindungsgemäßen Zubereitung lösen können, sondern in fester bzw. kristalliner Form vorliegen. Diese so gearteten Peelingsubstanzen kommen bei Anwendung während des Dusch- oder Waschvorgangs mit Wasser in Kontakt, beispielsweise beim Abduschen oder Abwaschen des Schaums und der Reinigungszubereitung am Ende des Reinigungsvorgangs. Hierdurch ist ein Auflösen der Zucker- und/oder Salzkristalle gewährleistet, was zu einem abnehmenden Peelingeffekt führt. Dies führt aber gleichzeitig auch dazu, dass keine oder praktisch keine Rückstände der Peelingsubstanzen in Waschbecken oder Behandlungsvorrichtungen zurückbleiben. Praktisch keine Rückstände bedeutet, dass in einzelnen wenigen Fällen äußerst geringe Spuren der Reinigungszubereitung nach dem Waschvorgang auf Waschtischen oder Anwendungseinrichtungen sichtbar sind, die jedoch auf einfache Weise durch Abspülen oder Wegwischen entfernbar sind.

Die erfindungsgemäß einsetzbaren Zucker können beispielsweise Mono-, Di- oder Oligosaccharide sein. Mono- oder Disaccharide können beispielsweise Saccharose, Glukose oder Fruktose sein, wobei Saccharose ganz besonders bevorzugt ist. Der Gehalt an Zucker beträgt 15 bis 45 Gew. %, bevorzugt 25 bis 35 Gew. %, bezogen auf das Gesamtgewicht der Zubereitung.

Die Salze in den erfindungsgemäßen Zubereitungen sollen hautverträglich und im Milieu der Reinigungszubereitungen geeignet sein, feste Strukturen, bevorzugt Kristalle zu bilden. Dies können beispielsweise Alkalisalze sein, wie Natriumchlorid, Kaliumchlorid, Natriumsulfat und/oder Kaliumsulfat, wobei Natriumchlorid ganz besonders bevorzugt ist. Die Salze in den erfindungsgemäßen Zubereitungen können ebenfalls Erdalkalisalze sein, wie beispielsweise Magnesiumchlorid und/oder Magnesiumsulfat. Der Gehalt der Salze beträgt 0 bis 35 Gew.-%, bevorzugt 0 bis 15 Gew.-%, bezogen auf das Gesamtgewicht der Zubereitung. Es gibt also auch vorteilhafte erfindungsgemäße Ausführungsformen, die keine Salze enthalten.

Die unlöslichen Peelingmittel können beispielsweise vorteilhaft aus der Gruppe Polyethylen, Tonerde, Sand, Kunststoffpartikel, zerstoßene oder zermahlene Kerne von Walnussschalen, Aprikosen-, Pfirsich- und/oder Mandelkernen gewählt werden.

Die Viskosität dieser Peelingsprodukte wurde mit einem deformationsgesteuerten Rotationsrheometer ARES 6 der Firma TA Instruments durchgeführt. Die Messung erfolgte mittels Rate Sweep Test, bei dem die Scherrate stufenförmig von 0,1 bis 1000 pro Sekunde mit 5 Messpunkten pro Dekade erhöht wird. Hierbei wird vor jedem Messpunkt während einer Wartezeit von 15 Sekunden die jeweilige Scherrate gehalten und in weiteren 5 Sekunden Messdaten erfasst, aus denen dann die Software den Messpunkt ermittelt. Als Geometrie kam ein Platte-Platte-Messsystem zum Einsatz mit einem Durchmesser von 25 mm und einem Messspalt von 1 mm (auf-grund der Peelingpartikel). Die Temperatur wurde mittels eines Peltiertemperiersystems an der unteren Messplatte auf 25°C gehalten. Beim Befüllen wurde der Rand 0,1 mm vor dem Erreichen des Messspalts bereinigt. Direkt nach dem Befüllen wurde vor dem Test eine Wartezeit von 2 Minuten zur Temperierung eingehalten. Die Viskosität wurde bei einer Scherrate von 10 pro Sekunde ausgewertet.

Folgende Herstellungsweise hat sich als vorteilhaft erwiesen:
Aufschmelzen der Antioxidantien und festen Fette in den flüssigen Ölen bei 50 bis 60 °C,
Abkühlen auf etwa 45°C,
unter Rühren (Blattrührer) Zugabe der Tensidmischung und des Parfüms,
Rühren bis die Mischung Raumtemperatur erreicht hat,
Zugabe der Peelingmischung.

### Beispiele:

| **Handelsname/Hersteller** | **INCI** | **OELPELL 131** | **OELPEEL 13 3** | **OELPEEL 151** | **OELPEEL 152** |
|---|---|---|---|---|---|
| **Sojaöl*,** Gustav Heess | Glycine Soja Oil + Ricinus Communis Seed Oil + Propyl Gallate | 10,12 | 10,12 | 12,62 | 8,00 |
| **Jonol CP**, Technochemie | BHT | 0,03 | 0,03 | 0,03 | 0,03 |
| **Akogel Plus,** AAK | Hydrogenated vegetable oil | 15,00 | 15,00 | 17,50 | 22,00 |
| **Glycerin** | Glycerin | 0,00 | 6,00 | 0,00 | 0,00 |
| **Zetesol 100**, Zschimmer&Schwarz | MIPA Laureth Sulfate + Laureth-4 + Cocamide DEA | 38,55 | 38,55 | 38,55 | 38,67 |
| **Parfum** | | 1,30 | 1,30 | 1,30 | 1,30 |
| **Sucrose** | | 30,00 | 25,00 | 25,00 | 25,00 |
| **Hydrated Silica** | | 5,00 | 4,00 | 5,00 | 5,00 |

| | | | | | |
|---|---|---|---|---|---|
| *99,919% Glycine Soja (Soybean) Oil 0,07% Ricinus Communis (Castor) Seed Oil 0,01% Propyl Gallate 0,001 % Citric Acid | | | | | |

### Weitere Ausführungsbeispiele:

| **Handelsname/Hersteller** | **INCI** | **Bsp.1** | **Bsp.2** | **Bsp.3** | **Bsp.4** | **Bsp.4** | **Bsp.5** | **Bsp.6** |
|---|---|---|---|---|---|---|---|---|
| **Sojaöl***, Gustav Heess | Glycine Soja Oil + Ricinus Communis Seed Oil + Propyl Gallate | 13% | 5% | 15,5% | 10% | 3,1% | 4% | 20% |
| Ri**cinusöl**, Gustav Heess | Ricinus Communis Seed Oil | | 2% | | | | 4% | |
| **Jonol CP**, Technochemie | BHT | 0,05% | 0,05% | 0,05% | 0,05% | 0,05% | 0,05% | 0,05% |
| **Akogel Plus,** AAK | Hydrogenated Vegetabe Oil | 17% | 21% | 17% | 15% | 22% | 14% | 20% |
| **VGB-22** ADM | Hydrogenated Rapeseed Oil | | | 1% | | | 2% | |
| **Glycerin** | | | | | 6% | | | 3% |
| **Zetesol 100**, Zschimmer&Schwarz | MIPA Laureth Sulfate + Laureth-4 + Cocamide DEA | 38,65% | 40,65% | 36,15% | 33,65% | 39% | 39,65 | 35,65 |
| **Parfum** | | 1,3% | 1,3% | 1,3% | 1,3% | 1,3% | 1,3% | 1,3% |
| **Sucrose** | | 25% | 25% | 30% | 30% | 30% | 25% | 10% |
| **Hydrated Silica** | | 5% | 5% | | 5% | 4% | | 5% |
| **Sodium Chloride** | | 0% | 0% | 0% | 0% | 0% | 10% | 5% |

| | | | | | | | | |
|---|---|---|---|---|---|---|---|---|
| *99,919% Glycine Soja (Soybean) Oil 0,07% Ricinus Communis (Castor) Seed Oil 0,01% Propyl Gallate 0,001% Citric Acid | | | | | | | | |

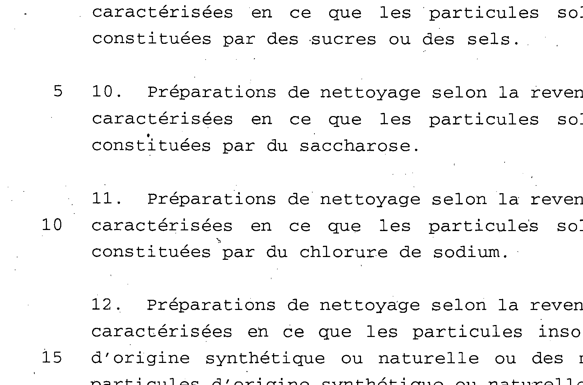

## Patentansprüche

1. Ölhaltige Reinigungszubereitungen in halbfester Form mit abrasiver Wirkung enthaltend
a) Wasser in einer Menge < 1,5 Gew.-%, bevorzugt < 0,2 Gew.-%, bezogen auf das Gesamtgewicht der Zubereitung,
b) Polyole mit einem Gehalt von < 10 Gew.-%, bezogen auf das Gesamtgewicht der Zubereitung,
c) Lipide, wobei die Lipide Gemische von wenigstens einem bei Raumtemperatur in flüssiger Form vorliegenden Lipid und von wenigstens einem bei Raumtemperatur in fester Form vorliegenden Lipid sind,
wobei das Gewichtsverhältnis des wenigstens einen bei Raumtemperatur festen Lipids zu dem wenigstens einen bei Raumtemperatur flüssigen Lipids 1:2 bis 7:1, bevorzugt 1:1 bis 3:1 beträgt,
wobei die bei Raumtemperatur in fester Form vorliegenden Lipide aus gehärteten Fetten und/oder Ölen bestehen und
die bei Raumtemperatur flüssigen Lipide pflanzliche Öle sind,
d) Gemische aus MIPA-Laurethsulfat, Laureth-4 und Kokosfettsäurediethanolamid als öllösliche Tenside,
e) abrasive Partikel, wobei die abrasiven Partikel feste Partikel sind, die lösliche oder unlösliche Partikel sind.

2. Reinigungszubereitungen nach Anspruch 1 **dadurch gekennzeichnet, dass** die Reinigungszubereitungen kein Wasser enthalten.

3. Reinigungszubereitungen nach wenigstens einem der vorstehenden Ansprüche **dadurch gekennzeichnet, dass** die Lipide mit einem Gehalt von 20 bis 60 Gew.-%, bevorzugt 25 bis 40 Gew.-%, bezogen auf das Gesamtgewicht der Zubereitung, vorliegen.

4. Reinigungszubereitungen nach wenigstens einem der vorstehenden Ansprüche **dadurch gekennzeichnet, dass** die bei Raumtemperatur festen Lipide ausgewählt werden aus der Gruppe Hydrogenated Vegetable Oil und Hydrogenated Rapeseed Oil.

5. Reinigungszubereitungen nach wenigstens einem der vorstehenden Ansprüche **dadurch gekennzeichnet, dass** die bei Raumtemperatur flüssigen Lipide ausgewählt werden aus der Gruppe Rizinusöl, Sojaöl und Öl aus Sonnenblumenkernen.

6. Reinigungszubereitungen nach wenigstens einem der vorstehenden Ansprüche **dadurch gekennzeichnet, dass** das Gewichtsverhältnis von Lipiden zu öllöslichen Tensiden 1: 3 bis 2:1 beträgt.

7. Reinigungszubereitungen nach wenigstens einem der vorstehenden Ansprüche **dadurch gekennzeichnet, dass** die Polyole Propylenglycol und/oder Glycerin sind.

8. Reinigungszubereitungen nach wenigstens einem der vorstehenden Ansprüche **dadurch gekennzeichnet, dass** die Polyole mit einem Gehalt von 0 bis 10 Gew.-%, bevorzugt 0 bis 6,0 Gew.-%, bezogen auf das Gesamtgewicht der Zubereitung, vorliegen.

9. Reinigungszubereitungen nach Anspruch 1 **dadurch gekennzeichnet, dass** die löslichen Partikel aus Zuckern oder Salzen bestehen.

10. Reinigungszubereitungen nach Anspruch 9 **dadurch gekennzeichnet, dass** die löslichen Partikel aus Saccharose bestehen.

11. Reinigungszubereitungen nach Anspruch 9 **dadurch gekennzeichnet, dass** die löslichen Partikel aus Natriumchlorid bestehen.

12. Reinigungszubereitungen nach Anspruch 1 **dadurch gekennzeichnet, dass** die unlöslichen Partikel synthetischen oder natürlichen Ursprungs oder Mischungen von Partikeln synthetischen oder natürlichen Ursprungs sind.

13. Reinigungszubereitungen nach wenigstens einem der vorstehenden Ansprüche **dadurch gekennzeichnet, dass** die Reinigungszubereitungen frei sind von polymeren Strukturanten und/oder Polyamidgelbildnern
wobei die polymeren Strukturanten Polymere sind, die als Struktureinheit (Monomer) wenigstens Acrylsäure und/oder Methacrylsäure und/oder Derivate der Acrylsäure und/oder Methacrylsäure enthalten.

14. Reinigungszubereitungen nach wenigstens einem der vorstehenden Ansprüche **dadurch gekennzeichnet, dass** die Reinigungszubereitungen frei sind von Konservierungsmitteln und/oder stabilisierenden Substanzen.

15. Reinigungszubereitungen nach wenigstens einem der vorstehenden Ansprüche **dadurch gekennzeichnet, dass** die Reinigungszubereitungen frei sind von Farbstoffen.

## Claims

1. Oil-containing cleaning preparations in semi-solid form having an abrasive effect and containing
a) water in an amount < 1.5 wt.%, preferably < 0.2 wt.%, relative to the total weight of the preparation,
b) polyols in a content of < 10 wt.%, relative to the total weight of the preparation,
c) lipids, the lipids being mixtures of at least one lipid that is in liquid form at room temperature and at least one lipid that is in solid form at room temperature,
the weight ratio of the at least one lipid that is solid at room temperature to the at least one lipid that is liquid at room temperature being 1:2 to 7:1, preferably 1:1 to 3:1,
the lipids in solid form at room temperature consisting of hydrogenated fats and/or oils, and the lipids in liquid form at room temperature being vegetable oils,
d) mixtures of MIPA-laureth sulfate, laureth-4 and coconut fatty acid diethanolamide as oil-soluble surfactants,
e) abrasive particles, the abrasive particles being solid particles that are soluble or insoluble particles.

2. Cleaning preparations according to claim 1, **characterized in that** the cleaning preparations contain no water.

3. Cleaning preparations according to at least one of the preceding claims, **characterized in that** the lipids are present in a content of 20 to 60 wt.%, preferably 25 to 40 wt.%, relative to the total weight of the preparation.

4. Cleaning preparations according to at least one of the preceding claims, **characterized in that** the lipids that are solid at room temperature are selected from the group comprising hydrogenated vegetable oil and hydrogenated rapeseed oil.

5. Cleaning preparations according to at least one of the preceding claims, **characterized in that** the lipids that are liquid at room temperature are selected from the group comprising castor oil, soya oil and sunflower seed oil.

6. Cleaning preparations according to at least one of the preceding claims, **characterized in that** the weight ratio of lipids to oil-soluble surfactants is 1:3 to 2:1.

7. Cleaning preparations according to at least one of the preceding claims, **characterized in that** the polyols are propylene glycol and/or glycerol.

8. Cleaning preparations according to at least one of the preceding claims, **characterized in that** the polyols are present in a content of 0 to 10 wt.%, preferably 0 to 6 wt.%, relative to the total weight of the composition.

9. Cleaning preparations according to claim 1, **characterized in that** the soluble particles consist of sugars or salts.

10. Cleaning preparations according to claim 9, **characterized in that** the soluble particles consist of sucrose.

11. Cleaning preparations according to claim 9, **characterized in that** the soluble particles consist of sodium chloride.

12. Cleaning preparations according to claim 1, **characterized in that** the insoluble particles are of synthetic or natural origin or are mixtures of particles of synthetic or natural origin.

13. Cleaning preparations according to at least one of the preceding claims, **characterized in that** the cleaning preparations are free from polymeric structuring agents and/or polyamide gelling agents, polymeric structuring agents being polymers containing as structural unit (monomer) at least acrylic acid and/or methacrylic acid and/or derivatives of acrylic acid and/or methacrylic acid.

14. Cleaning preparations according to at least one of the preceding claims, **characterized in that** the cleaning preparations are free from preservatives and/or stabilising substances.

15. Cleaning preparations according to at least one of the preceding claims, **characterized in that** the cleaning preparations are free from colourants.

## Revendications

1. Préparations de nettoyage contenant de l'huile sous forme semi-solide à effet abrasif, contenant :
a) de l'eau en une quantité < 1,5 % en poids, de préférence < 0,2 % en poids, par rapport au poids total de la préparation,
b) des polyols en une teneur < 10 % en poids, par rapport au poids total de la préparation,
c) des lipides, les lipides étant des mélanges d'au moins un lipide présent sous forme liquide à température ambiante et d'au moins un lipide présent sous forme solide à température ambiante,
le rapport en poids entre ledit au moins un lipide solide à température ambiante et ledit au moins un lipide liquide à température ambiante étant de 1:2 à 7:1, de préférence de 1:1 à 3:1,
les lipides présents sous forme solide à température ambiante étant constitués par des matières grasses et/ou des huiles durcies, et
les lipides liquides à température ambiante étant des huiles végétales;
d) des mélanges de MIPA-laureth sulfate, de laureth-4 et de diéthanolamide d'acide gras de coco en tant que tensioactifs solubles dans les huiles,
e) des particules abrasives, les particules abrasives étant des particules solides, qui sont des particules solubles ou insolubles.

2. Préparations de nettoyage selon la revendication 1, **caractérisées en ce que** les préparations de nettoyage ne contiennent pas d'eau.

3. Préparations de nettoyage selon au moins l'une quelconque des revendications précédentes, **caractérisées en ce que** les lipides sont présents en une teneur de 20 à 60 % en poids, de préférence de 25 à 40 % en poids, par rapport au poids total de la préparation.

4. Préparations de nettoyage selon au moins l'une quelconque des revendications précédentes, **caractérisées en ce que** les lipides solides à température ambiante sont choisis dans le groupe constitué par l'huile végétale hydrogénée et l'huile de colza hydrogénée.

5. Préparations de nettoyage selon au moins l'une quelconque des revendications précédentes, **caractérisées en ce que** les lipides liquides à température ambiante sont choisis dans le groupe constitué par l'huile de ricin, l'huile de soja et l'huile de tournesol.

6. Préparations de nettoyage selon au moins l'une quelconque des revendications précédentes, **caractérisées en ce que** le rapport en poids entre les lipides et les tensioactifs solubles dans les huiles est de 1:3 à 2:1.

7. Préparations de nettoyage selon au moins l'une quelconque des revendications précédentes, **caractérisées en ce que** les polyols sont le propylène glycol et/ou la glycérine.

8. Préparations de nettoyage selon au moins l'une quelconque des revendications précédentes, **caractérisées en ce que** les polyols sont présents en une teneur de 0 à 10 % en poids, de préférence de 0 à 6,0 % en poids, par rapport au poids total de la préparation.

9. Préparations de nettoyage selon la revendication 1, **caractérisées en ce que** les particules solubles sont constituées par des sucres ou des sels.

10. Préparations de nettoyage selon la revendication 9, **caractérisées en ce que** les particules solubles sont constituées par du saccharose.

11. Préparations de nettoyage selon la revendication 9, **caractérisées en ce que** les particules solubles sont constituées par du chlorure de sodium.

12. Préparations de nettoyage selon la revendication 1, **caractérisées en ce que** les particules insolubles sont d'origine synthétique ou naturelle ou des mélanges de particules d'origine synthétique ou naturelle.

13. Préparations de nettoyage selon au moins l'une quelconque des revendications précédentes, **caractérisées en ce que** les préparations de nettoyage sont exemptes de structurants polymères et/ou de gélifiants polyamide, les structurants polymères étant des polymères qui contiennent en tant qu'unité structurale (monomère) au moins de l'acide acrylique et/ou de l'acide méthacrylique et/ou des dérivés de l'acide acrylique et/ou de l'acide méthacrylique.

14. Préparations de nettoyage selon au moins l'une quelconque des revendications précédentes, **caractérisées en ce que** les préparations de nettoyage sont exemptes de conservateurs et/ou de substances stabilisantes.

15. Préparations de nettoyage selon au moins l'une quelconque des revendications précédentes, **caractérisées en ce que** les préparations de nettoyage sont exemptes de colorants.
